# FASCICULE DE BREVET EUROPEEN

(11) **EP 1 291 004 B1**
(45) Date de publication et mention de la délivrance du brevet: **04.10.2006**
(21) Numéro de dépôt: 02292138.1
(22) Date de dépôt: 29.08.2002
(51) Int. Cl.: A61K 8/33, A61K 8/73, A61Q 5/06

(54) **Composition coiffante aérosol à base de carboxyalkylalkylcellulose**
Haarfrisieraerosol auf Basis von Carboxyalkylalkylcellulose
Hair-styling aerosol composition based on carboxyalkyl-alkylcellulose

(30) Priorité: 31.08.2001 FR 0111343
(43) Date de publication de la demande: 12.03.2003
(73) Titulaire: L'ORÉAL, 75008 Paris (FR)
(72) Inventeur: Pataut, Françoise, 75017 Paris (FR); De Carvalho, Raquel, 92110 Clichy (FR)
(74) Mandataire: Casalonga, Axel

(56) Documents cités:
- EP-A- 1 114 637
- US-A- 4 784 848
- US-A- 5 030 443
- DATABASE CHEMICAL ABSTRACTS [en ligne] retrieved from STN Database accession no. 135: 66017 XP002205350 & WO 2001 045656 A (LION CORP.)
- DATABASE CHEMICAL ABSTRACTS [en ligne] retrieved from STN Database accession no. 133: 268594 XP002205351 & JP 2000 273485 A (NISSHIN CHEMICAL K.K.) 3 octobre 2000 (2000-10-03)

## Description

La présente invention concerne une composition de coiffage conditionnée dans un dispositif aérosol, contenant, en tant que polymère fixant, au moins une carboxyalkylalkylcellulose, ainsi qu'un procédé de coiffage utilisant une telle composition.

On connaît dans le domaine du coiffage un très grand nombre de polymères fixants, en particulier des polymères anioniques ou non ioniques, susceptibles d'être conditionnés, sous forme dissoute dans un milieu cosmétiquement acceptable, dans un dispositif aérosol.

Un grand nombre de ces polymères utilisés habituellement, lorsqu'ils présentent un pouvoir coiffant important, confèrent toutefois à la chevelure un aspect rigide, figé et peu naturel qui est de moins en moins recherché par les utilisateurs.

Le document US-A-5 030 443 décrit ainsi des compositions pour la mise en plis contenant un alginate, et éventuellement des dérivés de la cellulose pour modifier la viscosité de ces compositions.

Cet aspect figé est en partie dû à la température de transition vitreuse élevée du polymère lui ôtant toute plasticité à température ambiante, mais également au fait que le polymère fixant, une fois sec, colle les cheveux les uns aux autres, leur conférant ainsi un aspect de "casque". L'aspect figé de la coiffure ne disparaît qu'après brossage ou lavage des cheveux.

Un autre problème que pose la formulation de polymères fixants dans des dispositifs aérosols est lié à la viscosité des phases liquides à vaporiser. En effet, il est généralement souhaitable de formuler le polymère sous forme dissoute dans le milieu liquide. Or, la sélection et l'utilisation de bons solvants pour les polymères fixants implique le plus souvent une viscosité élevée du milieu liquide qui se traduit par une pulvérisation grossière, un risque de bouchage des valves et une répartition irrégulière de la solution sur les cheveux.

La demanderesse a découvert que l'utilisation d'une famille particulière de dérivés carboxylés d'éthers de cellulose connus, en tant que polymères fixants dans des compositions de coiffage conditionnées dans un dispositif aérosol permettait de surmonter les problèmes indiqués ci-dessus, c'est-à-dire de préparer des solutions de polymères fixants ayant des viscosités relativement faibles, compatibles avec de bonnes propriétés de pulvérisation.

Par ailleurs, la demanderesse a constaté que les dérivés carboxylés d'éthers de cellulose décrits plus en détail ci-après, lorsqu'on les utilise en tant que polymères fixants, confèrent aux cheveux une tenue satisfaisante résistant aux mouvements habituels de la tête et des cheveux et à des coups de vent, mais qui pouvait facilement être modulée par des sollicitations mécaniques modérées, par exemple par simple passage des mains dans les cheveux.

Ce type de fixation "friable", dû à des liens entre fibres différents de ceux créés par des polymères fixants habituels, présente donc l'avantage de conférer aux cheveux traités une tenue relativement souple et durable sans pour autant créer l'impression de rigidité observée habituellement pour les polymères fixants habituels.

La présente invention a par conséquent pour objet une composition de coiffage, conditionnée dans un dispositif aérosol, comprenant
- une phase liquide contenant, à l'état dissous dans un milieu liquide cosmétiquement acceptable, au moins un polymère filmogène fixant choisi parmi les carboxyalkylalkylcelluloses, et
- a moins un agent propulseur.

La présente invention a également pour objet un procédé de coiffage comprenant la pulvérisation d'une telle composition de coiffage sur les cheveux et l'évaporation du milieu liquide cosmétiquement acceptable.

Les carboxyalkylalkylcelluloses utilisées en tant que polymères fixants dans les compositions de coiffage de la présente invention sont connues en tant que telles. Elles sont préparées par exemple par carboxyalkylation partielle de cellulose suivie d'une alkylation du dérivé carboxylé obtenu.

Elles correspondent de préférence à des composés avec répétition de motifs de formule (I) 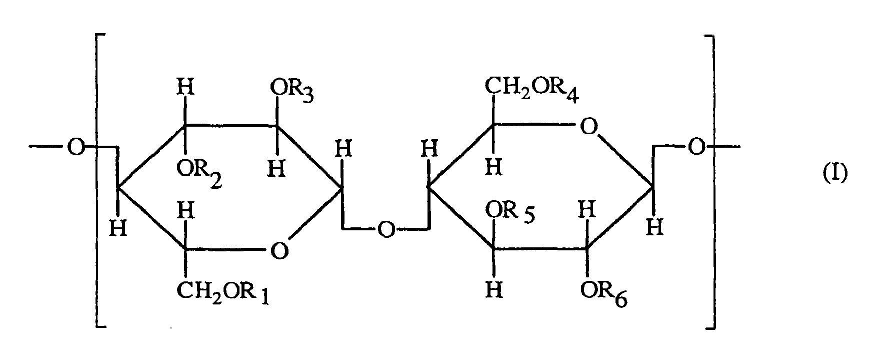 dans laquelle
R₁ à R₆ représentent indépendamment l'un de l'autre un atome d'hydrogène, un groupe alkyle en C₁₋₄ ou un groupe carboxy-(alkyle en C₁₋₄), et
le degré de polymérisation moyen étant de préférence compis entre 30 et 300,
et les sels cosmétiquement acceptables d'une telle molécule.

Comme indiqué ci-dessus, les carboxyalkylalkylcelluloses sont de préférence à l'état dissout dans la phase liquide des compositions coiffantes de la présente invention. La solubilité des carboxyalkylalkylcelluloses, c'est-à-dire leur aptitude à former, en présence de solvants, des milieux macroscopiquement homogènes, transparents, dépend de leurs taux de substitution, c'est-à-dire du nombre moyen de groupes carboxyalkyle et de groupes alkyle par motif de glucose, mais également de la masse moléculaire de ces polymères et du taux de neutralisation des groupes acide carboxylique.

Les carboxyalkylalkylcelluloses sous forme acide, non neutralisée, sont généralement insolubles ou difficilement solubles dans l'eau, mais présentent une solubilité satisfaisante dans des solvants organiques polaires tels que les alcools inférieurs, comme l'éthanol, l'isopropanol ou l'acétate d'éthyle, et en particulier dans des mélanges eau/alcool.
La solubilité dans l'eau de ces dérivés de cellulose peut être augmentée facilement par neutralisation des fonctions acide carboxylique par une base organique ou minérale cosmétiquement acceptable, choisie par exemple parmi les hydroxydes de métaux alcalins, en particulier l'hydroxyde de sodium ou de potassium, et les amines organiques, en particulier le 2-aminométhylpropanol.

Les carboxyalkylalkylcelluloses de l'invention sont de préférence utilisées sous forme partiellement ou totalement neutralisée par addition d'une base.

Dans un mode de réalisation préféré de la présente invention, la carboxyalkylalkylcellulose est une carboxyméthyléthylcellulose (CMEC), c'est-à-dire une carboxyalkylalkylcellulose de formule (I) dans laquelle au moins un des substituants R₁ à R₆ représente un atome d'hydrogène, au moins un des substituants R₁ à R₆ représente un groupe éthyle et au moins un des substituants représente un groupe carboxyméthyle.

Les carboxyméthyléthylcelluloses utilisées dans les compositions de coiffage de la présente invention ont de préférence une teneur en groupes carboxyméthyle comprise entre 8 et 16 % en poids, ce qui correspond en moyenne à peu près à un groupe carboxyméthyle pour deux motifs de glucose, et une teneur en groupes éthyle comprise entre 30 et 45 % en poids, ce qui équivaut à un taux de substitution voisin de 3.

On peut citer à titre d'exemple de produits commerciaux les carboxyméthyléthylcelluloses commercialisées par la société SANYO CHEMICAL.

Le milieu liquide cosmétiquement acceptable dans lequel sont introduites les carboxyalkylalkylcelluloses englobe par exemple l'eau et les solvants organiques cosmétiquement acceptables, ayant de préférence une volatilité supérieure à l'eau, tels que les alcools inférieurs, par exemple l'éthanol ou l'isopropanol, l'acétone, la méthyléthylcétone, le dichlorométhane ou l'acétate d'éthyle, ainsi que leurs mélanges.
Le milieu liquide cosmétiquement acceptable est de préférence un milieu aqueux, alcoolique ou hydroalcoolique, et en particulier un milieu hydroalcoolique contenant au moins 15 % en poids d'eau, de préférence de 15 à 90 % en poids d'eau, et en particulier de 20 à 80% en poids d'eau.

La phase liquide, également appelée "jus", des compositions de coiffage de la présente invention doit avoir une concentration minimale en carboxyalkylalkylcellulose permettant le dépôt d'une quantité suffisante de polymère fixant.
D'une manière générale, il s'est avéré qu'une concentration de carboxyalkylalkylcellulose dans la phase liquide comprise dans l'intervalle allant de 0,1 à 20 % en poids, et de préférence de 0,2 à 10 % en poids, et encore plus préférentiellement de 0,5 à 5%, convenait bien à l'invention.

On peut conditionner les compositions de coiffage de la présente invention dans des dispositifs aérosols en présence de n'importe quel agent propulseur usuellement employé pour la préparation de compositions aérosols. On utilisera de préférence des agents propulseurs non solubles ou partiellement solubles dans la phase liquide tels que le diméthyléther, les alcanes en C₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅.

Le rapport en poids de la phase liquide à l'agent propulseur des compositions aérosol de la présente invention est de préférence compris entre 20/80 et 80/20 et en particulier entre 30/70 et 70/30.

Les compositions de coiffage de la présente invention peuvent contenir - en plus d'une ou de plusieurs carboxyalkylalkylcelluloses - un ou plusieurs autres polymères fixants filmogènes connus anioniques , non ioniques, amphotères ou cationiques, ces copolymères fixants étant à l'état dissous ou dispersé (latex).

Toutefois, dans un mode de réalisation préféré des compositions de coiffage de la présente invention, les carboxyalkylalkylcelluloses sont les seuls polymères fixants présents et ne sont pas associées à un ou plusieurs autres polymères filmogènes fixants.
Les compositions de coiffage de la présente invention peuvent contenir en outre un ou plusieurs additifs cosmétiques ou de formulation utilisés habituellement dans le domaine cosmétique. On peut citer à titre de tels additifs, par exemple des filtres UV, des parfums, des agents conservateurs, des pigments et colorants, des agents solubilisants, des agents anti-mousse, des vitamines, des agents conditioneurs tels que des silicones solubles, dispersées ou microdispersée, des particules organiques ou minérales, synthétiques ou non, ou des agents tensioactifs.
Bien entendu l'homme du métier veillera à choisir ce ou ces éventuels additifs complémentaires et/ou leurs quantités de manière à ce que les propriétés avantageuses intrinsèques à l'invention ne soient pas altérées par la ou les adjonctions envisagées.

Les compositions coiffage de la présente invention sont de préférence des laques pour cheveux.

L'exemple de formulation illustre la présente invention sans cependant la limiter.

### Exemple

### Spray aérosol

On dissout 2,5 g de carbométhyléthylcellulose (Sanyo Chemical) dans un mélange de 43,6 g d'eau et de 19 g d'éthanol, on neutralise avec 0,44 g de 2-aminométhylpropanol, et on conditionne le jus ainsi obtenu avec 35 g de diméthyléther (agent propulseur) dans un dispositif aérosol.

Cette laque, contenant moins de 55 % en volume de composés organiques volatils, se laisse vaporiser facilement, confère aux cheveux une tenue satisfaisante et durable, qui peut être facilement modelée par simple passage des mains.

## Revendications

1. Composition de coiffage conditionnée dans un dispositif aérosol comprenant
• une phase liquide contenant, à l'état dissous dans un milieu liquide cosmétiquement acceptable, au moins un polymère filmogène fixant choisi parmi les carboxyalkylalkylcelluloses, et
• au moins un agent propulseur.

2. Composition de coiffage selon la revendication 1, **caractérisée par le fait que** les carboxyalkylalkylcelluloses correspondent à des composés avec répétition de motifs de formule (I) 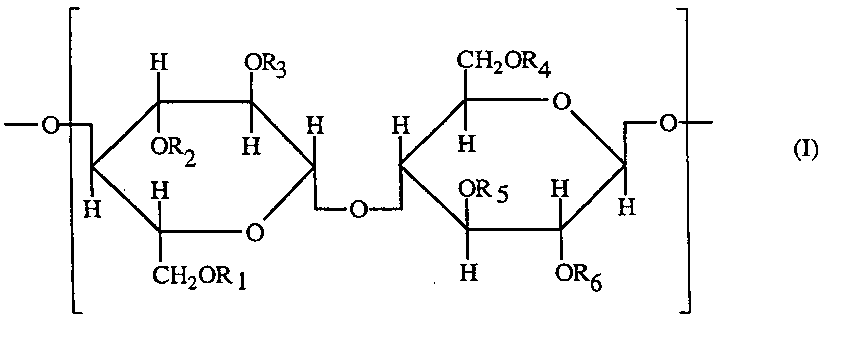 dans laquelle
R₁ à R₆ représentent indépendamment l'un de l'autre un atome d'hydrogène, un groupe alkyle en C₁₋₄ ou un groupe carboxy-(alkyle en C₁₋₄),
et leurs sels cosmétiquement acceptables.

3. Composition de coiffage selon la revendication 1 ou 2, **caractérisée par le fait que** le degré de polymérisation moyen est compris entre 30 et 300.

4. Composition de coiffage selon l'une quelconque des revendications précédentes, **caractérisée par le fait que** la carboxyalkylalkylcellulose est une carboxyméthyléthylcellulose.

5. Composition de coiffage selon la revendication 4, **caractérisé par le fait que** la carboxyméthyléthylcellulose a une teneur en groupes carboxyméthyle comprise entre 8 et 16 % en poids et une teneur en groupes éthyle compris entre 30 et 45 % en poids.

6. Composition de coiffage selon l'une quelconque des revendications précédentes, **caractérisée par le fait que** le milieu cosmétiquement acceptable est un milieu aqueux, alcoolique ou hydroalcoolique.

7. Composition de coiffage selon la revendication 6, **caractérisée par le fait que** le milieu hydroalcoolique contient au moins 15% en poids d'eau, de préférence de 15 à 90% en poids d'eau.

8. Composition de coiffage selon l'une quelconque des revendications précédentes, **caractérisée par le fait que** la concentration de la carboxyalkylalkylcellulose dans la phase liquide est comprise entre 0,1 et 20% en poids, de préférence entre 0,2 et 10% en poids.

9. Composition de coiffage selon l'une quelconque des revendications précédentes, **caractérisée par le fait qu'**elle ne contient pas de polymères filmogènes fixants différents des carboxyalkylalkylcelluloses de formule (I).

10. Composition de coiffage selon l'une quelconque des revendications précédentes, **caractérisée par le fait que** le ou les agents propulseurs sont choisis parmi le diméthyléther, les alcanes en C₃₋₅, le 1,1-difluoroéthane, les mélangs de diméthyléther et d'alcanes en C₃₋₅, et les mélanges de 1,1-difluoroéthane et de diméthyléther et/ou d'alcanes.

11. Composition de coiffage selon l'une quelconque des revendications précédentes, **caractérisée par le fait que** le rapport en poids de la phase liquide à l'agent propulseur est compris entre 20/80 et 80/20, de préférence entre 30/70 et 70/30.

12. Composition de coiffage selon l'une quelconque des revendications précédentes, **caractérisée par le fait qu'**il s'agit d'une laque pour cheveux.

13. Procédé de coiffage comprenant la pulvérisation d'une composition de coiffage, conditionnée dans un dispositif aérosol, selon l'une quelconque des revendications précédentes, sur les cheveux et l'évaporation du milieu liquide cosmétiquement acceptable.

## Claims

1. Styling composition packaged in an aerosol device, comprising
• a liquid phase containing, in dissolved form in a cosmetically acceptable liquid medium, at least one film-forming fixing polymer chosen from carboxyalkylalkylcelluloses, and
• at least one propellant.

2. Styling composition according to Claim 1, **characterized in that** the carboxyalkylalkylcelluloses correspond to compounds with a repetition of units of formula (I) 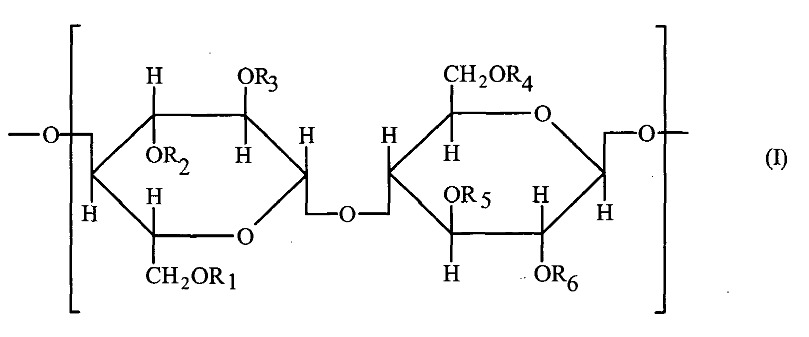 in which
R₁ to R₆ represent, independently of each other, a hydrogen atom, a C₁-C₄ alkyl group or a carboxy(C₁₋C₄ alkyl) group,
and the cosmetically acceptable salts thereof.

3. Styling composition according to Claim 1 or 2, **characterized in that** the average degree of polymerization is between 30 and 300.

4. Styling composition according to any one of the preceding claims, **characterized in that** the carboxyalkylalkylcellulose is a carboxymethylethylcellulose.

5. Styling composition according to Claim 4, **characterized in that** the carboxymethylethylcellulose has a carboxymethyl group content of between 8% and 16% by weight and an ethyl group content of between 30% and 45% by weight.

6. Styling composition according to any one of the preceding claims, **characterized in that** the cosmetically acceptable medium is an aqueous, alcoholic or aqueous-alcoholic medium.

7. Styling composition according to Claim 6, **characterized in that** the aqueous-alcoholic medium contains at least 15% by weight of water and preferably from 15% to 90% by weight of water.

8. Styling composition according to any one of the preceding claims, **characterized in that** the carboxyalkylalkylcellulose concentration in the liquid phase is between 0.1% and 20% by weight and preferably between 0.2% and 10% by weight.

9. Styling composition according to any one of the preceding claims, **characterized in that** it does not contain any film-forming fixing polymers other than the carboxyalkylalkylcelluloses of formula (I).

10. Styling composition according to any one of the preceding claims, **characterized in that** the propellant (s) is (are) chosen from dimethyl ether, C₃-C₅ alkanes, 1,1-difluoroethane, mixtures of dimethyl ether and of C₃-C₅ alkanes, and mixtures of 1,1-difluoroethane and of dimethyl ether and/or of alkanes.

11. Styling composition according to any one of the preceding claims, **characterized in that** the weight ratio of the liquid phase to the propellant is between 20/80 and 80/20 and preferably between 30/70 and 70/30.

12. Styling composition according to any one of the preceding claims, **characterized in that** it is a hair lacquer.

13. Styling process that involves spraying a styling composition, packaged in an aerosol device, according to any one of the preceding claims, onto the hair and evaporating the cosmetically acceptable liquid medium.

## Patentansprüche

1. Zusammensetzung für die Frisurengestaltung, die in einer Aerosolvorrichtung konfektioniert ist, die enthält:
• eine flüssige Phase, die in einem kosmetisch akzeptablen, flüssigen Medium in gelöstem Zustand mindestens ein filmbildendes fixierendes Polymer enthält, das unter den Carboxyalkylalkylcellulosen ausgewählt ist, und
• mindestens ein Treibmittel.

2. Zusammensetzung für die Frisurengestaltung nach Anspruch 1, **dadurch gekennzeichnet, dass** die Carboxyalkylalkylcellulosen Verbindungen mit Wiederholungseinheiten der Formel (I): 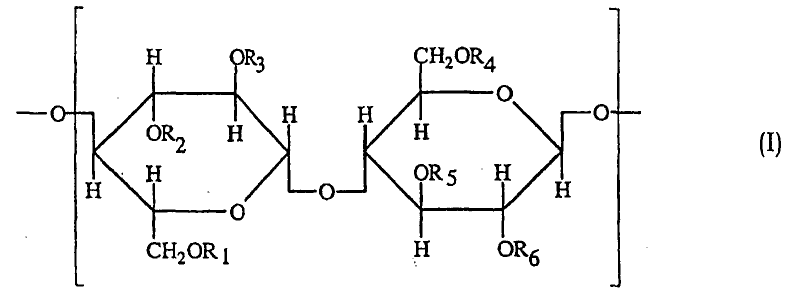 worin bedeuten:
die Gruppen R₁ bis R₆ unabhängig voneinander ein Wasserstoffatom, eine C₁₋₄-Alkylgruppe oder eine Carboxyalkyl(C₁₋₄)gruppe und deren kosmetisch akzeptablen Salzen entsprechen.

3. Zusammensetzung für die Frisurengestaltung nach Anspruch 1 oder 2, **dadurch gekennzeichnet, dass** der mittlere Polymerisationsgrad im Bereich von 30 bis 300 liegt.

4. Zusammensetzung für die Frisurengestaltung nach einem der vorgehenden Ansprüche, **dadurch gekennzeichnet, dass** die Carboxyalkylalkylcellulose die Carboxymethylethylcellulose ist.

5. Zusammensetzung für die Frisurengestaltung nach Anspruch 4, **dadurch gekennzeichnet, dass** die Carboxymethylethylcellulose einen Gehalt an Carboxymethylgruppen von 8 bis 16 Gew.-% und einen Gehalt an Ethylgruppen von 30 bis 45 Gew.-% aufweist.

6. Zusammensetzung für die Frisurengestaltung nach einem der vorhergehenden Ansprüche, **dadurch gekennzeichnet, dass** das kosmetisch akzeptable Medium ein wässriges, alkoholisches oder wässrig-alkoholisches Medium ist.

7. Zusammensetzung für die Frisurengestaltung nach Anspruch 6, **dadurch gekennzeichnet, dass** das wässrig-alkoholische Medium mindestens 15 Gew.-% Wasser und vorzugsweise 15 bis 90 Gew.-% Wasser enthält.

8. Zusammensetzung für die Frisurengestaltung nach einem der vorhergehenden Ansprüche, **dadurch gekennzeichnet, dass** die Konzentration der Carboxyalkylalkylcellulose in der flüssigen Phase im Bereich von 0,1 bis 20 Gew.-% und vorzugsweise 0,2 bis 10 Gew.-% liegt.

9. Zusammensetzung für die Frisurengestaltung nach einem der vorhergehenden Ansprüche, **dadurch gekennzeichnet, dass** sie keine fixierenden filmbildenden Polymere enthält, die von den Carboxyalkylalkylcellulosen der Formel (I) verschieden sind.

10. Zusammensetzung für die Frisurengestaltung nach einem der vorhergehenden Ansprüche, **dadurch gekennzeichnet, dass** das oder die Treibmittel unter Dimethylether, C₃₋₅-Alkanen, 1,1-Difluorethan, Gemischen von Dimethylether und C₃₋₅-Alkanen und Gemischen von 1,1-Difluorethan und Dimethylether und/oder Alkanen ausgewählt sind.

11. Zusammensetzung für die Frisurengestaltung nach einem der vorhergehenden Ansprüche, **dadurch gekennzeichnet, dass** das Gewichtsverhältnis der flüssigen Phase und des Treibmittels im Bereich von 20/80 bis 80/20 und vorzugsweise 30/70 bis 70/30 liegt.

12. Zusammensetzung für die Frisurengestaltung nach einem der vorhergehenden Ansprüche, **dadurch gekennzeichnet, dass** es sich um einen Haarlack handelt.

13. Verfahren für die Frisurengestaltung, das das Sprühen einer in einer Aerosolvorrichtung konfektionierten Zusammensetzung für die Frisurengestaltung nach einem der vorhergehenden Ansprüche auf die Haare und das Verdampfen des kosmetisch akzeptablen flüssigen Mediums umfasst.
